# EUROPEAN PATENT APPLICATION

(11) **EP 2 719 457 A1**
(43) Date of publication of application: **16.04.2014**
(21) Application number: 12187738.5
(22) Date of filing: 09.10.2012
(51) Int. Cl.: B01L 3/00, C12M 1/00

(54) **Multiwell plate device and method of use**

(71) Applicant: Qiagen GmbH, 40724 Hilden (DE)
(72) Inventor: Singer, Thorsten, Dr., 42699 Solingen (DE)
(74) Representative: Gille Hrabal

(57) **Abstract**

The invention relates to a multiwell plate device, in particular for purification of a sample, preferably a genomic DNA or RNA from cells, tissue, and blood as well as a method thereof.

The multiwell plate device comprises a tray base and a multiwell insert element having a plurality of bottomless wells formed therein. The multiwell insert element is detachably attached to the tray base, in particular so as to minimize or substantially prevent the fluid communication between adjacent wells when attached.

## Description

The present invention relates to a multiwell plate device. In particular, the invention relates to a multiwell plate device for purification of a sample, preferably a DNA or RNA from cells, tissue, and blood as well as a method of use.

Multiwell plate devices serve a broad spectrum of laboratory uses. They are also known as microplates or microtitre/microtiter plates, and allow efficient sample processing and analysis due to the plurality of wells provided thereon. In general, multiwell plates have standard dimensions for providing for example 6, 24, 96, 384 or 1536 wells arranged in a rectangular array. The standard for multiwell plates has been established by the Society of Biomolecular Sciences (SBS Standard).

A typical application for multiwell plates is the purification of "molecules of interest", e.g. nucleic acids or proteins from biological samples, especially genomic DNA from cells, tissue, and blood. The general procedure involves the steps of lysis, washing, and elution. For a convenient and rapid automated procedure, workstations (e.g. BioSprint 96 from QIAGEN) are available which enable an automated sample preparation and eliminate time-consuming manual processing steps. In particular, magnetic-particle technology is used for attracting or releasing molecules of interest present in the wells of a multiwell plate. This can be done by adding a suspension with magnetic particles to a sample, whereby target molecules bind to the magnetic particles. Depending on the target material, the purification of a sample may require a sequential processing in different buffer solutions. Normally magnetic rods are used to transfer the magnetic particles from tube to tube or well to well in order to perform a rapid purification procedure and yield a substantially pure target molecule (e.g. genomic DNA). The purified target molecule such as a DNA or RNA is suited for sensitive downstream analyses, for example PCR analyses.

Robotic instruments are available for performing automated processing of multiwell plates. Such automated processes include, for example, deposition of biological materials, addition or removal of reagents, washing, scanning and analysis.

Although automatic robotic processing has been rendered possible by the standard structure of multiwell plates, the filling of multiwell plates with buffer solutions and the like is still time-consuming as generally each well is filled individually by an automated dispenser. Even if the (automated) dispenser comprises several parallel multi-channel pipettes, several filling procedures may be necessary depending on the well volume. In addition, sophisticated machinery for automated dispensing or filling of the multiwell plates is necessary. This increases the footprint of the overall workstation and requires cost-intensive machinery.

The multiwell plates may also be filled manually with pipettes such as multi-channel pipettes. The manual workflow is tedious and time-consuming due to the labor-intensive procedure, in particular when the multiwell plates have a high number of wells provided thereon (e.g. 384-well or 1536-well plates). The precise and efficient dispensing and filling of multiwell plates with a buffer solution therefore poses a challenge.

Document US 2009/0069200 A1 reveals a microtiter plate having an array of wells, wherein an array of subwells is provided within each well in the array of wells. A reservoir beneath each array of subwells is in fluid communication with all the subwells in the array of subwells. In an embodiment, the microtiter plate comprises a porous separator between the subwell array and reservoir to keep analyte beads from entering the reservoir below the subwell array. The arrangement of the microtiter plate particularly with the porous separator leads to an elaborate structure with constraints regarding the manufacturing of the microtiter plate. In addition, the microtiter plate does not prevent the fluid communication between adjacent subwells.

Unless indicated otherwise, the aforementioned features can be combined separately or in any combination with the subject matter of the following invention.

The present invention seeks to provide an improved multiwell plate device.

In accordance with the present invention, a multiwell plate device as defined in claim 1 is provided. Further advantageous embodiments are outlined in the dependent claims.

In order to solve the problem the multiwell plate device comprises a tray base and a multiwell insert element having a plurality of bottomless wells formed therein. The multiwell insert element is detachably attached to the tray base, in particular so as to minimize or substantially prevent the fluid communication between adjacent wells when attached.

The detachable attachment of the multiwell insert element facilitates the rapid preparation of a multiwell plate device. In particular, the arrangement of the multiwell plate device renders it possible that a buffer solution or the like can rapidly be distributed and filled in the plurality of wells formed in the multiwell insert element. In use, the tray base of the multiwell plate device can be filled with a buffer solution and subsequently the multiwell insert element can be attached to the tray base to provide a multiwell plate device with a plurality of wells. The attachment is preferably carried out by a vertical movement of the multiwell insert element, thereby dividing the inside space of the tray base into a plurality of wells. The buffer solution contained in the tray base is accordingly distributed over the wells of the assembled multiwell plate device enabling an efficient filling, preparation and processing of the multiwell plate device.

In addition, it is possible to provide various well configurations in the multiwell plate device by changing the spacing, arrangement, and/or dimension of the wells formed in the multiwell insert element. Depending on the application, the multiwell insert element may be adapted to comprise a suitable well volume. The multiwell plate device can thereby be adapted to various applications as the possible combinations of tray base and multiwell insert element are manifold.

Preferably the volume of the wells formed in the multiwell insert element is equal. This allows for a rapid, parallel sample preparation or processing using the multiwell plate device.

The attachment of the multiwell insert element in the tray base preferably minimizes or substantially prevents a fluid communication between adjacent wells when attached. A marginal fluid communication for example a diffusion of the contained fluid between adjacent wells is generally negligible as the molecules of interest e.g. genomic DNA molecules are preferably purified using magnetic particles, which are particularly adapted for a specific binding property so that the target molecules bind to the magnetic particles. In this way, it is ensured that the target molecules are substantially contained in each well by the magnetic particles. It is particularly advantageous to minimize the fluid communication between adjacent wells. This avoids any potential cross-contamination therefore increasing the purity of the isolated target molecules to such an extent that the sensitivity in downstream analyses (e.g. PCR analyses) can be further increased.

In an embodiment, the multiwell insert element is dimensioned to be substantially coextensive with the inner boundary of the tray base. This ensures that the multiwell insert element fits securely in the tray base, providing a sufficient fit or seal between the multiwell insert element and the inside wall of the tray base.

In an embodiment, the depth of the wells formed in the multiwell insert element is at least 5 mm, preferably at least 10 mm, and particularly preferred at least 25 mm. The depth of the wells is dimensioned in such a way that a magnetic separation, in particular using magnetic rods, is rendered possible. The magnetic separation is generally carried out by an up and down movement of a magnetic rod to collect and/or release magnetic particles into tubes or wells. The adequate depth of the wells provided in the multiwell plate device ensures a rapid and efficient processing of magnetic particles, especially using automated workstations. In an embodiment, the tray base, in particular the inner tray base wall and/or the tray base bottom, comprises at least one notch and/or protrusion configured to engage with the multiwell insert element. When the tray base comprises a protrusion, the multiwell insert element may feature a corresponding notch. The notch and/or protrusion are preferably formed integrally in the tray base. Additionally or alternatively, the multiwell insert element may also comprise a notch and/or protrusion to be configured to engage with the tray base. This facilitates the engagement of the tray base with the multiwell insert element. The alignment of the protrusion and/or notch is particularly adapted to the wells formed in the multiwell insert element to secure and enhance the fit of the multiwell insert element in the tray base. With regard to the wells of the attached multiwell plate device, this also minimizes the fluid communication between adjacent wells. It is particularly preferred that the dimensions of the notch and/or protrusion are selected in such a manner that the fluid communication between adjacent wells is substantially prevented when the multiwell insert element is attached to the tray base. In particular, the engagement between the tray base and the multiwell insert element via the notch and/or protrusion is configured to be form-fitting and/or friction-locked.

For additional ease in assembling the multiwell plate device, the notch and/or protrusion in the tray base may be provided in the form of rails. Correspondingly, the multiwell insert element may be configured to engage with the rails provided in the tray base to allow a simple attachment of the multiwell insert element while substantially preventing a fluid communication between adjacent wells. To provide a flexible tray base, the rails in the tray base may be arranged for different configurations of the multiwell insert element. The rails in the tray base may accordingly be used depending on the configuration of the multiwell insert element. This allows for a flexible usage of the tray base with different multiwell insert elements.

The multiwell plate device is preferably configured to be disposable. In this regard part or all of the components of the multiwell plate device may be designed for a single use. This helps minimize the risk of cross-contamination and enables a reliable purification workflow using the multiwell plate device.

In order to further prevent the fluid communication between adjacent wells, at least one sealing element may be provided between the tray base and the multiwell insert element. In particular, the sealing element may be provided in the form of a gasket or packing. This ensures that the fluid communication between adjacent wells is prevented, leading to a higher purity of the isolated molecules, thereby also increasing the sensitivity for downstream analyses.

In an embodiment, the tray base and/or the multiwell insert element comprises at least one fastening element and/or retention element to lock or retain the multiwell insert element in the tray base, particularly in a releasable manner. The fastening element and/or retention element may be realized by a snap-fit, snap joint, or snap-on connection. This enables the secure attachment of the multiwell insert element in the tray base, preventing unintentional loosening or releasing of the components.

In order to allow a convenient handling, the multiwell insert element comprises at least one handling element. The handling element is preferably part of the multiwell insert element. It is also possible to attach a handling element to the multiwell insert element by a fastening means such as a clip, bolt, clamp or the like. The handling element may be formed or shaped as a grip, pull handle, or hook-shaped element. The handling element on the multiwell insert element facilitates the convenient detachment.

The tray base bottom may be formed to be flat, concave, or tapered. The concave or tapered designs are particularly advantageous for applications requiring a transfer of the contents in the wells, for example by manual or robotic pipetting.

In an embodiment, the dimension of the multiwell plate device, the well dimension, and/or the well spacing conform to the SBS standard. The dimensions for typical multiwell plates conforming to the SBS standard are approximately 85 mm (width) x 1 25 mm (length) with the wells arranged in a standardized format depending on the total number of wells. This ensures that the multiwell plate device is compatible for use in workstations or automated sample preparation systems specifically adapted to handle SBS conforming multiwell plates. In conjunction with the time saving preparation of the multiwell plate devices this enables a rapid and efficient automated sample preparation, in particular for the purification of genomic DNA using magnetic-particle technology. In addition, the assembly of the multiwell plate device ensures a particularly flexible and versatile multiwell plate. Overall a streamlined workflow for purification of a sample such as a genomic DNA is rendered possible, which particularly reduces the preparation time for filling the multiwell plate device with a buffer solution.

In an embodiment, the outer wells formed in the multiwell insert element are provided with at least one open vertical wall portion, in particular such that the inner portion of the tray base wall constitutes a peripheral boundary for the outer wells. Wells arranged in the corners of the multiwell insert element may comprise two open vertical wall portions. When the multiwell insert element is attached to the tray base, the inner portion of the tray base wall constitutes the wall boundary for the outer wells formed in the multiwell insert element. This simplifies the design structure of the multiwell insert element and facilitates an easy attachment of the multiwell insert element in the tray base. In particular, the buffer solution contained in the tray base is easily distributed by the structure and filled into the wells of assembled multiwell plate device.

The tray base and/or the multiwell insert element can be made from any moldable material, such as a plastic polymer. Suitable preferred materials include plastic polymers such as polyethylene, polypropylene, polystyrol, thermoplastic elastomer and the like. The tray base and/or the multiwell insert element is preferably constructed from a single piece of material, The tray base and/or the multiwell insert element may be molded, for example by injection molding. This enables a fast and cost-efficient manufacturing of the multiwell plate device.

A further aspect of the present invention provides a kit for purification of a genomic DNA or RNA from cells, tissue, and blood. The kit provides a multiwell plate device as described above and at least one buffer solution, such as a washing or elution buffer. With regard to the multiwell plate device, reference may be made to the above description.

Embodiments of the present invention will be described, by way of example, with reference to the accompanying drawings in which:
- Fig. 1: is a perspective view of a tray base for a multiwell plate device, and
- Fig. 2: is a perspective view of a multiwell insert element.

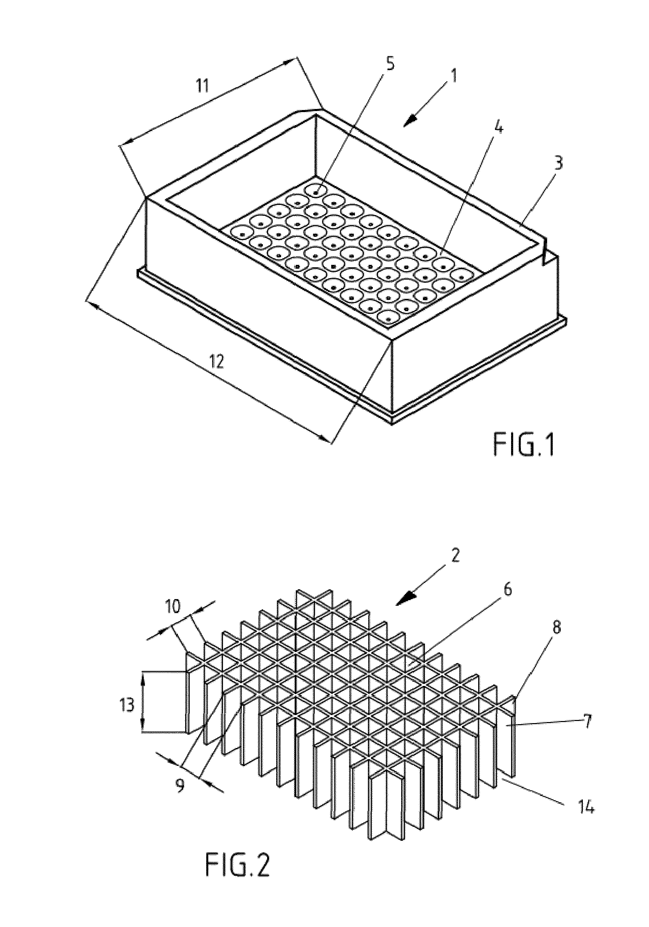

Referring to Fig. 1, a tray base 1 for a multiwell plate device comprises a tray base wall 3 and a tray base bottom 4 enclosing an inside space for receiving a multiwell insert element 2. It can be seen that the multiwell insert element 2 shown in Fig. 2 can be detachably attached to the tray base 1. In use, the tray base 1 is at first filled with a buffer solution or the like. Afterwards, the multiwell insert element 2 is inserted into the tray base 1 in a vertical direction. The buffer solution in the tray base is thereby equally distributed into the wells 6 formed in the multiwell insert element 2.

The tray base bottom 4 may comprise recesses 5 such as concave recesses as shown in Fig. 1. Alternatively, the recesses 5 may be formed with a tapered or flat shape. The alignment of the recesses 5 in the tray base bottom 4 may correspond to the alignment of the wells 6 formed in the multiwell insert element 2. The length 12 of the overall tray base 1 may be approximately 125 mm. The width 11 of the overall tray base 1 may be approximately 86 mm. In particular, the dimensions of the multiwell plate device, the well dimensions such as the longitudinal 9 or transversal 10 spacing and/or the well spacing conform to the SBS standard for multiwell plates in order to ensure the compatibility with workstations or automated sample preparation systems adapted to handle SBS conforming multiwell plates.

The well depth 13 of the wells formed in the multiwell insert element 2 may be at least 5 mm, preferably at least 10 mm, and particularly preferred at least 25 mm. This ensures an adequate depth for carrying out a magnetic separation using for example magnetic rods to attract and/or release magnetic particles to transfer target molecules from well to well 6.

In this embodiment, the multiwell insert element 2 consists of a plurality of parallel longitudinal walls 7 and a plurality of parallel transversal walls 7. The longitudinal and transversal walls are aligned to perpendicularly cross each other to form the wells 6 therein. The spacing between the walls, namely the longitudinal spacing 9 and the transversal spacing 10 is preferably adapted to conform to the well spacing of the SBS standard for multiwell plates. The dimensions of the wells 6 are preferably configured to enclose a volume in each well of approximately 300 to 900. When the multiwell plate device has substantially less than 96 wells provided thereon, the dimensions of the wells 6 may be configured to enclose a volume in each well of approximately 900 - 10000 µl. The outer wells formed in the multiwell insert element 2 are preferably provided with a partially open vertical wall portion 14. The wells provided in the corners may comprise an open vertical wall portion that is greater than the rest of the multiwell insert element 2. When the multiwell insert element 2 is attached to the tray base 1 , the inner portion of the tray base wall 3 constitutes the wall for the outer wells formed in the multiwell insert element 2. The structure of the multiwell insert element 2 facilitates the attachment to the tray base 1 and the equal distribution of a buffer solution contained in the tray base into the plurality of wells formed in the multiwell insert element 2.

It can be further seen from Fig. 2 that the longitudinal spacing 9 and the transversal spacing 10 of the wells 6 is preferably equal to provide wells with equal volumes. Depending on the application, the wells 6 may also enclose differing volumes.

It can be understood that the multiwell plate device provides a simple and cost-efficient solution for distributing a buffer solution in a plurality of wells and thereby enabling an efficient sample preparation in a multiwell plate device. The multiwell plate device may be advantageously combined with automated workstations, robotic handling systems, and robotic sample preparation systems, further reducing the overall hands-on time for preparing and processing a multiwell plate device. In particular, the multiwell plate device allows an efficient parallel sample preparation in a flexible manner by allowing the easy configuration of wells provided in the multiwell plate device and facilitating the rapid distribution of a buffer solution or the like in the wells of a multiwell plate device in one step. The multiwell plate device can be adapted to provide a configurable number of wells and/or well volumes, making it suitable for a multitude of applications, especially for the rapid purification of target molecules such as nucleic acids or proteins from biological samples.

### Reference numerals:

- 1: Tray base
- 2: Multiwell insert element
- 3: Tray base wall
- 4: Tray base bottom
- 5: Recess
- 6: Well
- 7: Longitudinal wall
- 8: Transversal wall
- 9: Longitudinal spacing
- 10: Transversal spacing
- 11: Width of 1
- 12: Length of 1
- 13: Well depth
- 14: Open vertical wall portion

## Claims

1. A multiwell plate device comprising a tray base (1), a multiwell insert element (2) having a plurality of bottomless wells (6) formed therein, **characterized in that** the multiwell insert element (2) is detachably attached to the tray base (1), in particular so as to minimize or substantially prevent the fluid communication between adjacent wells (6) when attached.

2. A multiwell plate device according to the preceding claim, wherein the multiwell insert element (2) is dimensioned to be substantially coextensive with the inner boundary of the tray base (1).

3. A multiwell plate device according to any preceding claim, wherein the tray base bottom (4) constitutes the bottom for the wells (6) formed in the multiwell insert element (2) when attached.

4. A multiwell plate device according to any preceding claim, wherein the multiwell insert element (2) comprises a plurality of longitudinal and transversal walls (7, 8), which in particular are configured to perpendicularly cross each other.

5. A multiwell plate device according to any preceding claim, wherein the well depth (13) is at least 5 mm, preferably at least 10 mm, and particularly preferred at least 25 mm.

6. A multiwell plate device according to any preceding claim, wherein the tray base (1), especially the tray base wall (3) and/or the tray base bottom (4), comprises at least one notch and/or protrusion configured to engage with portions of the multiwell insert element (2).

7. A multiwell plate device according to any preceding claim, wherein at least one sealing element is provided between the tray base (1) and the multiwell insert element (2), in particular in the form of a gasket or packing.

8. A multiwell plate device according to any preceding claim, wherein the tray base (1) and/or the multiwell insert element (2) comprises at least one fastening element and/or retention element to releasably lock or retain the multiwell insert element (2) in the tray base (1).

9. A multiwell plate device according to any preceding claim, wherein the multiwell insert element (2) comprises at least one handling element so as to facilitate the attachment and/or detachment of the multiwell insert element (2).

10. A multiwell plate device according to any preceding claim, wherein the tray base bottom (4) comprises recesses (5), which in particular correspond to the wells (6) formed in the multiwell insert element (2).

11. A multiwell plate device according to any preceding claim, wherein the dimensions of the multiwell plate (1), the dimensions of the wells (6), and/or the spacing of the wells (6) conform to the SBS standard.

12. A multiwell plate device according to any preceding claim, wherein the outer wells (6) formed in the multiwell insert element (2) are provided with at least one open vertical wall portion (14), in particular such that the inner portion of the tray base wall (3) constitutes a peripheral boundary for the outer wells (6).

13. A multiwell plate device according to any preceding claim, wherein the tray base (1) and/or the multiwell insert element (2) comprises a material selected from the group of plastic polymer, polyethylene, polypropylene, polystyrol, thermoplastic elastomer and the like, in particular such that the tray base (1) and/or the multiwell insert element (2) is constructed from a single piece of material.

14. A kit for purification of a sample, especially of a DNA or RNA from cells, tissues, and blood, incorporating a multiwell plate device according to any preceding claim, and at least one buffer solution.

15. A method of using a multiwell plate device according to any preceding claim for purification of a sample, comprising:
filling the tray base (1) with a buffer solution, particularly with a washing or elution buffer, and
attaching the multiwell insert element (2) to the tray base (1) in a vertical direction to distribute the buffer solution in the wells formed in the multiwell insert element (2),
and wherein in particular magnetic separation, especially using magnetic rods, is used to attract and/or release target molecules from the wells (6).
